# EUROPEAN PATENT SPECIFICATION

(11) **EP 1 729 718 B1**
(45) Date of publication and mention of the grant of the patent: **13.08.2008**
(21) Application number: 04812253.5
(22) Date of filing: 24.11.2004
(51) Int. Cl.: A61K 8/365, A61Q 7/02

(54) **REDUCTION OF HAIR GROWTH BY APPLYING AN AGONIST OF PROSTAGLANDIN DP-RECEPTOR**
VERRINGERUNG DES HAARWACHSTUMS DURCH ANLEGEN EINES AGONISTEN DES PROSTAGLANDIN-DP-REZEPTORS
REDUCTION DE LA POUSSE DES POILS GRACE A L'APPLICATION D'UN AGONISTE DE RECEPTEUR DP DE PROSTAGLANDINE

(30) Priority: 25.11.2003 US 721118
(43) Date of publication of application: 13.12.2006
(73) Proprietor: The Gillette Company, Boston, Massachusetts 02199 (US)
(72) Inventor: HWANG, Cheng, S., Framingham, MA 01702 (US); AHLUWALIA, Gurpreet, S., Wellesley, MA 02482 (US); SHANDER, Douglas, Acton, MA 01720 (US)
(74) Representative: Wilding, Richard Alan
(86) International application number: PCT/US2004/039693
(87) International publication number: WO 2005/051335

(56) References cited:
- EP-A- 1 358 868
- WO-A-96/26712
- WO-A-98/08089
- US-A- 4 562 204
- US-A- 6 093 748

## Description

The invention relates to reducing hair growth in mammals, particularly for cosmetic purposes.

A main function of mammalian hair is to provide environmental protection. However, that function has largely been lost in humans, in whom hair is kept or removed from various parts of the body essentially for cosmetic reasons. For example, it is generally preferred to have hair on the scalp but not on the face.

Various procedures have been employed to remove unwanted hair, including shaving, electrolysis, depilatory creams or lotions, waxing, plucking, and therapeutic antiandrogens. These conventional procedures generally have drawbacks associated with them. Shaving, for instance, can cause nicks and cuts, and can leave a perception of an increase in the rate of hair regrowth. Shaving also can leave an undesirable stubble. Electrolysis, on the other hand, can keep a treated area free of hair for prolonged periods of time, but can be expensive, painful, and sometimes leaves scarring. Depilatory creams, though very effective, typically are not recommended for frequent use due to their high irritancy potential. Waxing and plucking can cause pain, discomfort, and poor removal of short hair. Finally, antiandrogens -- which have been used to treat female hirsutism -- can have unwanted side effects.

It has previously been disclosed that the rate and character of hair growth can be altered by applying to the skin inhibitors of certain enzymes. These inhibitors include inhibitors of 5-alpha reductase (see, for example, Breuer et al., U.S. Pat. No. 4,885,289); ornithine decarboxylase (see, for example, Shander, U.S. Pat. No. 4,720,489), S-adenosylmethionine decarboxylase (see, for example Shander, U.S. patent 5,132,293); adenylosuccinate synthase (see, for example, Ahluwalia); U.S. Pat. No. 5,095,007); aspartate transcarbamylase (see, for example, Ahluwalia, U.S. Pat. No. 5,095,007); gamma-glutamyl transpeptidase (see, for example, Ahluwalia et al., U.S. Pat. No. 5,096,911); transglutaminase (see, for example, Shander, et al., U.S. Patent 5,143,925); L-asparagine synthetase (see, for example, Ahluwalia, U.S. Patent 5,444,090); 5-lipoxygenase (see, for example, Ahluwalia et al., U.S. Patent 6,239,170); cyclooxygenase (see, for example, Ahluwalia et al., U.S. Patent 6,248,751); nitric oxide synthase (see, for example Ahluwalia et al., U.S. Patent 5,468,476); ornithine aminotransferse (see, for example, Shander et al.,U.S. Patent 5,474,763); cysteine synthetic pathway enzymes including L-methionine S-adenosyltransferase. L-homocysteine S-methyl transferase, S-adenosyl homocysteine hydrolyase, cystathionine synthase and cystathionase (see, for example, Ahluwalia et al., U.S. Patent 5,455,234); cholesterol synthesis pathway enzymes including HMGCoA reductase and squalene synthetase (see, for example, Henry et al., U.S. Patent 5840752); protein kinase C (see, for example, Ahluwalia et al.,U.S. Patent 5,554,608); arginase (see, for example, Shander et al., U.S. Patent 5,728,736); matrix metalloproteinase (see for example Styczynski et al., U.S. Patent 5,962,466); DNA topoisomerase (see, for example Styczynski et al., U.S. Patent 6,037,326); aminoacyl-tRNA synthetase (see, for example, Henry et al., U.S. Patent 5,939,458); hypusine biosynthetic pathway enzymes including deoxyhypusine synthase and deoxyhypusine hydroxylase (see, for example, Styczynski et al. U.S. Patent 6,060,471); alkaline phosphatase (see, for example Styczynski et al., U.S. Patent 6,020,006); and protein-tyrosine kinase (see, for example Henry et al., U.S. Patent 6,121,269).

U.S. Patent 5,908,867 (Henry et al.) describes a method for reducing mammalian hair growth by inhibiting the formation of glycoproteins, proteglycans or glycosaminoglycans, for example by use of inhibitors of the synthesis of N-acetyl glucosamine-pyrophosphoryl-dolichyl, chondrotin sulfate, keratin sulfate, dermatan sulfate, heparan sulfate, heparin, hyaluronic acid, inhibitors of the formation of Glc₃Man₉-(GlcNAc)₂-PP-dolichol or glycosaminoglycan hyaluronic acid, inhibitors of the transfer of Glc₃Man₉-(GlcNAc)₂, inhibitors of the enzymes glucosidase I, glucosidase II, mannosidas I, mannosidase II, and β-galactosidase and compounds that affect the exocytosis of proteoglycans.

U.S. Patents 5,652,273, 5,824,665 and 6,218,435 (all issued to Henry et al.) describe ways of reducing mammalian hair growth by suppression of the metabolic pathway for conversion of glucose to acetyl-Co-A. This can be effected by, inter alia, inhibition of hexokinase, phosphofructokinase, aldolase, phosphoglycerate kinase, enolase, pyruvate kinase or pyruvate dehydrogenase or by an inhibitor of glucose transport.

Other methods for modulating hair growth include use of compounds that induce or activates conjugation of an androgen, for example as described by Styczynski et al., in U.S. Patent 5,958,946; use of compounds to increase cellular ceramide levels for example as described by Styczynski et al., in U.S. Patent 6,235,737; use of non-steroidal suppressors of angiogenesis, for example as described in Ahluwalia in U.S. Patent 6,093,748; use of catechin compounds, for example as described in U.S. Patents 5,674,477 and 5,776,442 (Ahluwalia); use of sulfhydryl reactive compounds for example as described in Shander et al. In U.S. Patent 5,411,991; and use of pantothenic acid or an analogue thereof, for example as described by Ahluwalia et al in U.S. Patent 5,364,885. PCT Publication WO03086331 (Ahluwalia et al) discloses that mammalian hair growth can be reduced by topical application of an inhibitor of fatty acid metabolism.

There are four primary prostaglandins (PGs), namely, PGE₂, PGF₂, PGI₂ and PGD₂. They are synthesized and released from cells as a result of various chemical or physical stimuli. The released prostaglandins act as local hormones on cells in the vicinity to exert a variety of actions such as inflammation and muscle contraction or relaxation. Each prostaglandin binds to specific members of a family of G protein-coupled prostaglandin receptors. The interaction between prostaglandin and receptor activates intracellular G protein signal transduction pathways that alter the levels of second messengers such as cAMP, Ca²⁺ and inositol phosphates. It is this alteration of the second messenger levels that are thought to evokes a cell response. The receptor(s) for each prostaglandin is coupled to a unique signal transduction pathway. The specificity is such that a minor diversity in a prostaglandin's chemical structure can result in as much as complete opposite effect even in the same tissue. For example, the prostaglandin PGD₂ causes relaxation of smooth muscle, whereas PGE₂ induces the contraction of smooth muscle.Chemically, prostaglandins contain a cyclopentane ring with the two-side chains alpha (-) and omaga (-). Prostaglandins are classified into prostaglandin types A through I based on modifications of the cyclopentane ring. However, the only naturally occurring prostaglandins are types D through I.

The cell receptors for the prostaglandins PGD₂, PGE₂, PGF_{2α}, and PGI₂ are designated as DP, EP, FP and IP, respectively. The EP class of receptors is further divided into four subtypes: EP1, EP2, EP3, and EP4. Because of the diversity in prostaglandin receptors, as well as the complexity of prostaglandin signaling pathways, it is generally accepted that the knowledge about the function of any one of the prostaglandin in a given tissue cannot be extrapolated to another prostaglandin in the same tissue, or the same prostaglandin in another tissue. Each prostaglandin has a unique activity profile not exactly overlapping with others, indicating that each prostaglandin has a specific site of action.

| Prostaglandin (PG) or Prostanoid | G-protein-coupled PG or Prostanoid receptor* |
|---|---|
| PGE₂ | EP (EP1, EP2, EP3 and EP4) |
| PGF_{2α} | FP |
| PGI₂ | IP |
| PGD₂ | DP |

| | |
|---|---|
| *The receptors are distinguished by their ligand-binding profiles, and the signal transduction pathways activated on ligand binding. | |

In one aspect, the invention provides a cosmetic method) of reducing unwanted mammalian (preferably human) hair growth by applying to the skin an agonist of prostaglandin DP-receptor in an amount effective to reduce hair growth. Preferably, the agonist interacts strongly with the prostaglandin DP-receptor. The unwanted hair growth may be undesirable from a cosmetic standpoint or may result, for example, from a disease or an abnormal condition (e.g., hirsutism).

In another aspect, the invention provides a method of reducing unwanted mammalian hair growth by applying to the skin a compound selected from the group consisting of prostaglandin D₂, analogs or derivatives of prostaglandin D₂, PGJ₂, or an analog of PGJ₂.

Typically, in practicing the aforementioned methods, the agonist/compound will be included in a topical composition along with a dermatologically or cosmetically acceptable vehicle. The topical compositions comprise a dermatologically or cosmetically acceptable vehicle and an agonist of prostaglandin DP receptor. The topical compositions comprise a dermatologically or cosmetically acceptable vehicle and (a) a compound selected from the group consisting of prostaglandin D₂, analogs or derivatives of prostaglandin D₂, PGJ₂, or an analog of PGJ₂; (b) a compound that activates DP receptor signal transmission pathway; and/or (c) a compound that inactivates prostaglandin D₂ metabolic pathway.

In addition, the present invention relates to the use of an agonist of prostaglandin DP-receptor for the manufacture of a therapeutic topical composition for reducing hair growth. Further, the present invention relates to the use of a compound for the manufacture of a therapeutic topical composition for reducing hair growth, wherein the compound is (a) a compound selected from the group consisting of prostaglandin D₂, analogs or derivatives of prostaglandin D₂, PGJ₂, or an analog of PGJ₂; (b) a compound that activates DP receptor signal transmission pathway; and/or or (c) a compound that inactivates prostaglandin D₂ metabolic pathway.

"Agonist of prostaglandin DP-receptor", as used herein, means a compound that activates prostaglandin DP receptor. An agonist that "interacts strongly" with the prostaglandin DP-receptor is one that that binds the receptor with such affinity that it elicits a response that is at least approximately comparable to (in magnitude) to that elicited by prostaglandin D₂. "DP receptor", as used herein, means the receptor is of the class that has the strongest affinity for PGD₂ of all the naturally occurring prostaglandins.

Specific compounds include both the compound itself and pharmacologically acceptable salts of the compound.

Other features and advantages of the invention may be apparent from the detailed description and from the claims.

An example of a preferred composition includes at least one agonist of prostaglandin DP-receptor in a cosmetically and/or dermatologically acceptable vehicle. The composition may be a solid, semi-solid, or liquid. The composition may be, for example, a cosmetic and dermatologic product in the form of an, for example, ointment, lotion, foam, cream, gel, or solution. The composition may also be in the form of a shaving preparation or an aftershave. The vehicle itself can be inert or it can possess cosmetic, physiological and/or pharmaceutical benefits of its own.

Examples of agonists of prostaglandin DP-receptor include prostaglandin D₂ analogs and prostaglandin D₂-sequential metabolites and their analogs.

Analogs of prostaglandin D₂ (also referred to as PGD₂) are known. For example, U.S. Pat. 4,203,924 describes 2-decarboxy-2-hydroxymethyl-deoxy-9,10-didehydro-PGD₂ analogs; U.S. Pat. 4,201,873 describes 9-deoxy-9,10-didehydro-PGD₂ analogs; U.S. Pat. 4,562,204 describes trans-delta2-prostaglandin D₂ derivatives; U.S. Pat. 3,878,239 describes preparation of other prostaglandin D₂ analogs; and U.S. Pat. 5,700,835 describes 3-oxa-D-prostaglandins. Other examples of prostaglandin D₂ analogs are disclosed EP 0 098 141 and EP 0 097 023.

Prostaglandin D₂ sequential metabolites and their analogs also are known.

Specific examples of agonists are provided in Tables 1 and 1 A.

**TABLE I**

| **Examples of PGD₂ analogs, PGD₂ sequential metabolites and their analogs** | |
|---|---|
| PGD₂ analog | 11-Deoxy-11-methylene PGD₂, 15(R)-15-methyl PGD₂,15(S)- |
| | 15-methyl PGD₂, 15-deoxy-Δ^{12,14}-PGD₂, 16,16-dimethyl-PGD₂, 17-phenyl trinor PGD₂, 9β-halogen-15-cyclohexylprostaglandin, 11α-halogen-15-cyclohexyl-prostaglandin, ZK118182, RS93520, RS93427, SQ27986, ZK110841, BW245C, BW246C, BW A868C, L644122, and L644698. |
| PGD₂ metabolites | 13, 14-Dihydro-15-keto PGD₂, PGJ₂, Δ¹²-PGJ₂, 15-deoxy-Δ^{12,14} |
| and PGJ₂ analogs | PGJ₂, 9,10-dihydro-15-deoxy-Δ^{12,14}-PGJ₂ |

**TABLE IA**

| | |
|---|---|
| **Chemical names of the PGD₂ analogs from Table I** | |
| ZK118182 | Acetic acid, [[(2Z)-4-[(1R,2R,3R,5R)-5-chloro-2-[(1E,3S)-3-cyclohexyl-3-hydroxy-1-propenyl]-3-hydroxycyclopentyl]-2-butenyl]oxy]- (9CI) |
| RS93520 | Butanoic acid, 4-[(1R,2R,3S,6R)-2-[(3S)-3-cyclohexyl-3-hydroxy-1-propynyl]-3-hydroxybicyclo[4.2.0]oct-7-ylidene]-, (4Z)- (9CI) |
| RS93427 | Butanoic acid, 4-[(1S,2S,3R,6S)-2-[(3S)-3-cyclohexyl-3-hydroxy-1-propynyl]-3-hydroxybicyclo[4.2.0]oct-7-ylidene]-, (4Z)-(9CI) |
| SQ27986 | 5-Heptenoic acid, 7-[(1S,2S,3S,4R)-3-[(1E,3S)-3-cyclohexyl-3-hydroxy-1-propenyl]-7-oxabicyclo[2.2.1]hept-2-yl]-, (5Z)- (9CI) |
| ZK110841 | 5-Heptenoic acid, 7-[(1R,2R,3R,5R)-5-chloro-2-[(1E,35)-3-cyclohexyl-3-hydroxy-1-propenyl]-3-hydroxycyclopentyl]-, (5Z)-(9CI) |
| BW245C | 4-Imidazolidineheptanoic acid, 3-[(3R)-3-cyclohexyl-3-hydroxypropyl]-2, 5-dioxo-, (4S)-rel- (9CI) |
| BW246C | (4R)-(3-[(3R,S)-3-Cyclohexyl-3-hydroxypropyl]-2,5-dioxo)-4-imidazolidineheptanoic acid |
| L44122 | Benzoic acid, 4-[3-[3-[2-(1-hydroxycyclohexyl)ethyl]-4-oxo-2-thiazolidinyl]propyl]- (9CI) |
| L44698 | Benzoic acid; 4-[3-[3-(3-hydroxyoctyl)-4-oxo-2-thiazolidinyl]propyl]- (9CI) |
| BWA868C | 4-Imidazolidineheptanoic acid, 3-[(2-cyclohexyl-2-hydroxyethyl)amino]-2,5-dioxo-1-(phenylmethyl)- (9CI) |

The composition may include more than one agonist of PGD₂. In addition, the composition may include one or more other types of hair growth reducing agents, such as those described in U.S. Pat. No. 4,885,289; U.S. Pat. No. 4,720,489; U.S. Pat. No. 5,132,293; U.S. Pat. 5,096,911; U.S. Pat. No. 5,095,007; U.S. Pat. No. 5,143,925; U.S. Pat. No. 5,328,686; U.S. Pat. No. 5,440,090; U.S. Pat. No. 5,364,885; U.S. Pat. No. 5,411,991; U.S. Pat. No. 5,648,394; U.S. Pat. No. 5,468,476; U.S. Pat. No. 5,475,763; U.S. Pat. No. 5,554,608; U.S. Pat. No. 5,674,477; U.S. Pat. No. 5,728,736; U.S. Pat. 5,652,273; WO 94/27586; WO 94/27563; and WO 98/03149, all of which are incorporated herein by reference.

The concentration of the agonist in the composition may be varied over a wide range up to a saturated solution, preferably from 0.1% to 30% by weight or even more; the reduction of hair growth increases as the amount of agonist applied increases per unit area of skin. The maximum amount effectively applied is limited only by the rate at which the agonist penetrates the skin. The effective amounts may range, for example, from 10 to 3000 micrograms or more per square centimeter of skin.

The vehicle can be inert or can possess cosmetic, physiological and/or pharmaceutical benefits of its own. Vehicles can be formulated with liquid or solid emollients, solvents, thickeners, humectants and/or powders. Emollients include stearyl alcohol, mink oil, cetyl alcohol, oleyl alcohol, isopropyl laurate, polyethylene glycol, petroleum jelly, palmitic acid, oleic acid, and myristyl myristate. Solvents include ethyl alcohol, isopropanol, acetone, diethylene glycol, ethylene glycol, dimethyl sulfoxide, and dimethyl formamide.

The composition optionally can include components that enhance the penetration of the agonist into the skin and/or to the site of action. Examples of penetration enhancers include urea, polyoxyethylene ethers (e.g., Brij-30 and Laureth-4), 3-hydroxy-3,7,11-trimethyl-1,6,10-dodecatriene, terpenes, cis-fatty acids (e.g., oleic acid, palmitoleic acid), acetone, laurocapram, dimethylsulfoxide, 2-pyrrolidone, oleyl alcohol, glyceryl-3-stearate, propan-2-ol, myristic acid isopropyl ester, cholesterol, and propylene glycol. A penetration enhancer can be added, for example, at concentrations of 0.1% to 20% or 0.5% to 5% by weight.

The composition also can be formulated to provide a reservoir within or on the surface of the skin to provide for a continual slow release of the agonist. The composition also may be formulated to evaporate slowly from the skin, allowing the agonist extra time to penetrate the skin.

A topical cream composition containing an agonist of PGD₂ may be prepared by mixing together water and all water soluble components in a mixing vessel- A. The pH is adjusted in a desired range from about 3.5 to 8.0. In order to achieve complete dissolution of ingredients the vessel temperature may be raised to up to 45°C. The selection of pH and temperature will depend on the stability of the agonist. The oil soluble components, except for the preservative and fragrance components, are mixed together in another container (B) and heated to up to 70°C to melt and mix the components. The heated contents of vessel B are poured into the water phase (container A) with brisk stirring. Mixing is continued for about 20 minutes. The preservative components are added at temperature of about 40°C. Stirring is continued until the temperature reaches about 25°C to yield a soft cream with a viscosity of 8,000 - 12,000 cps, or a desired viscosity. The fragrance components are added at about 25°C - 30°C while the contents are still being mixed and the viscosity has not yet built up to the desired range. If it is desired to increase the viscosity of the resulting emulsion, shear can be applied using a conventional homogenizer, for example a Silverson L4R homogenizer with a square hole high sheer screen. The topical composition can be fabricated by including the agonist in the water phase during formulation preparation or can be added after the formulation (vehicle) preparation has been completed. The agonist can also be added during any step of the vehicle preparation. The components of cream formulations are described in the examples below.

**EXAMPLE 1 (CREAM)**

| **INCI Name** | W/w (%) |
|---|---|
| DI Water | 61.00-75.00 |
| Agonist of PGD₂ | 1.00-15.00 |
| Mineral oil | 1.90 |
| Glyceryl stearate | 3.60 |
| PEG 100 stearate | 3.48 |
| Cetearyl alcohol | 2.59 |
| Ceteareth-20 | 2.13 |
| Dimethicone, 100 ct | 0.48 |
| Lipidure PMB^{a} | 3.00 |
| Advanced moisture complex^{b} | 5.00 |
| Stearyl alcohol | 1.42 |
| Preservative, fragrance and color pigment | Qs |
| **Total** | 100.00 |

| | |
|---|---|
| ^{a}polyquartinium-51 (Collaborative Labs, NY); ^{b}glycerin and water and sodium PCA and urea and trehalose and polyqauternium-51 and sodium hyaluronate (Collaborative Labs, NY). | |

**EXAMPLE 2 (CREAM)**

| **INCI Name** | w/w (%) |
|---|---|
| Agonist of PGD₂ | 0.5-15.00 |
| Glycerol (glycerin) | 0-5 |
| Isoceteth-20 | 3 - 7 |
| Glyceryl isostearate | 1.5 - 5 |
| Dicaprylyl ether | 3-15 |
| Glyceryl triacetate (triacetin) | 0.5 - 10 |
| Preservative, fragrance and color pigment | q.s. |
| Water | q.s. to 100.00 |

**EXAMPLE 3 (CREAM)**

| **INCI Name** | w/w (%) |
|---|---|
| Agonist of PGD₂ | 0.5 - 15.00 |
| Glycerol (glycerin) | 0 - 5 |
| Isoceteth-20 | 3-7 |
| Glyceryl isostearate | 1.5 - 5 |
| Dicaprylyl ether | 3-15 |
| 1-dodecyl-2-pyrrolidanone | 0.5-10% |
| Preservative, fragrance and color | q.s. |
| Water | to 100.00 |

**EXAMPLE 4 (CREAM)**

| **INCI Name** | w/w (%) |
|---|---|
| Water | 470 |
| Glyceryl stearate | 4 |
| PEG-100 | 4 |
| Cetearyl alcohol | 3 |
| Ceteareth-20 | 2.5 |
| Mineral oil | 2 |
| Stearyl alcohol | 2 |
| Dimethicone | 0.5 |
| Preservatives | 0.43 |
| 1-Dodecyl-2-pyrrolidanone | 1-10 |
| **Total** | 100.00 |

An agonist of PGD₂ is added to the example 4 formulation and mixed until solubilized.

**EXAMPLE 5 (CREAM)**

| **INCI Name** | w/w (%) |
|---|---|
| Water | 70-80 |
| Glyceryl stearate | 4 |
| PEG-100 | 4 |
| Cetearyl alcohol | 3 |
| Ceteareth-20 | 2.5 |
| Mineral oil | 2 |
| Stearyl alcohol | 2 |
| Dimethicone | 0.5 |
| Preservatives | 0.43 |
| Monocaprylate/Caprate (Estol 3601, Uniquema, NJ) | 1-10 |
| **Total** | 100.00 |

An agonist of PGD₂ is added to the example 5 formulation and mixed until solubilized.

**EXAMPLE 6 (CREAM)**

| **INCI Name** | w/w (%) |
|---|---|
| Water | 70-80 |
| Glyceryl stearate | 4 |
| PEG-100 | 4 |
| Cetearyl alcohol | 3 |
| Ceteareth-20 | 2.5 |
| Mineral oil | 2 |
| Stearyl alcohol | 2 |
| Dimethicone | 0.5 |
| Preservatives | 0.43 |
| cis Fatty acids | 1-10 |
| **Total** | 100.00 |

An agonist of PGD₂ is added to the example 6 formulation and mixed until solubilized.

**EXAMPLE 7 (CREAM)**

| **INCI Name** | w/w (%) |
|---|---|
| Water | 70-80% |
| Glyceryl stearate | 4 |
| PEG-100 | 4 |
| Cetearyl alcohol | 3 |
| Ceteareth-20 | 2.5 |
| Mineral oil | 2 |
| Stearyl alcohol | 2 |
| Dimethicone | 0.5 |
| Preservatives | 0.43 |
| Terpene(s) | 1-10 |
| **Total** | 100.00 |

An agonist of PGD₂ is added to the example 7 formulation and mixed until solubilized.

**EXAMPLE 8 (CREAM)**

| **INCI Name** | w/w (%) |
|---|---|
| Water | 70-80% |
| Glyceryl stearate | 4 |
| PEG-100 | 4 |
| Cetearyl alcohol | 3 |
| Ceteareth-20 | 2.5 |
| Mineral oil | 2 |
| Stearyl alcohol | 2 |
| Dimethicone | 0.5 |
| Preservatives | 0.43 |
| Polyoxyethylene sorbitans (tween) | 1-10 |
| **Total** | 100.00 |

An agonist of PGD₂ is added to the example 8 formulation and mixed until solubilized.

A hydroalcoholic formulation containing an agonist of PGD₂ is prepared by mixing the formulation components in a mixing vessel. The pH of the formulation is adjusted to a desired value in the range of 3.5 - 8.0. The pH adjustment can also be made to cause complete dissolution of the formulation ingredients. In addition, heating can be applied to up to 45°C, or even up to 70°C depending on the stability of the agonist to achieve dissolution of the formulation ingredients. The components of two hydroalcoholic formulations are listed below.

**EXAMPLE 9 (HYDRO-ALCOHOLIC)**

| **INCI Name** | w/w (%) |
|---|---|
| Water | 48.00 - 62.50 |
| An agonist of PGD₂ | 0.5-15.00 |
| Ethanol | 16.00 |
| Propylene glycol | 5.00 |
| Dipropylene glycol | 5.00 |
| Benzyl alcohol | 400 |
| Propylene carbonate | 2.00 |
| Captex-300^{a} | 5.00 |
| **Total** | 100.00 |

| | |
|---|---|
| ^{a}caprylic/capric triglyceride (Abitec Corp., OH). | |

**EXAMPLE 10 (HYDRO-ALCOHOLIC)**

| **INCI Name** | w/w (%) |
|---|---|
| Water | 53.00-67.9 |
| An agonist of PGD₂ | 0.1-15.00 |
| Ethanol | 16.00 |
| Propylene glycol | 5.00 |
| Dipropylene glycol dimethyl ether | 5.00 |
| Benzyl alcohol | 4.00 |
| Propylene carbonate | 2.00 |
| **Total** | 100.00 |

**EXAMPLE 11 (HYDRO-ALCOHOLIC)**

| **INCI Name** | w/w (%) |
|---|---|
| Ethanol (alcohol) | 80 |
| Water | 17.5 |
| Propylene glycol dipelargonate | 2.0 |
| Propylene glycol | 0.5 |
| **Total** | 100.00 |

An agonist of PGD₂ is added to the Example 11 formulation and mixed until solubilized.

The composition should be applied topically to a selected area of the body from which it is desired to reduce hair growth. For example, the composition can be applied to the face, particularly to the beard area of the face, i.e., the cheek, neck, upper lip, and chin. The composition also may be used as an adjunct to other methods of hair removal including shaving, waxing, mechanical epilation, chemical depilation, electrolysis and laser-assisted hair removal.

The composition can also be applied to the legs, arms, torso or armpits. The composition is particularly suitable for reducing the growth of unwanted hair in women having hirsutism or other conditions. In humans, the composition should be applied once or twice a day, or even more frequently, to achieve a perceived reduction in hair growth. Perception of reduced hair growth could occur as early as 24 hours or 48 hours (for instance, between normal shaving intervals) following use or could take up to, for example, three months. Reduction in hair growth is demonstrated when, for example, the rate of hair growth is slowed, the need for removal is reduced, the subject perceives less hair on the treated site, or quantitatively, when the weight of hair removed (i.e., hair mass) is reduced.

### HUMAN HAIR FOLLICLE GROWTH ASSAY

Human hair follicles in growth phase (anagen) were isolated from face-lift tissue (obtained from plastic surgeons) under dissecting scope using a scalpel and watchmakers forceps. The skin was sliced into thin strips exposing 2-3 rows of follicles that could readily be dissected. Follicles were placed into 0.5 ml William's E medium (Life Technologies, Gaithersburg, MD.) supplemented with 2 mM L-glutamine, 10 µg/ml insulin, 10 ng/ml hydrocortisone, 100 units of penicillin, 0.1 mg/ml streptomycin and 0.25 µg/ml amphotericin B. The follicles were incubated in 24-well plates (1 follicle/well) at 37°C in an atmosphere of 5% CO₂ and 95% air. Compounds are dissolved into dimethyl sulfoxide as 100-fold stock solution. The control hair follicles were treated with dimethyl sulfoxide without prostaglandin. The follicles were photographed in the 24-well plates under the dissecting scope at a power of 10X. Typically, image recordings were made on day 0 (day follicles were placed in culture), and again on day 7. The length of hair follicle was assessed using an image analysis software system. The growth of hair fiber was calculated by the subtracting the follicle length on day 0 from that determined on day 7.

PGD₂ and its analogs demonstrated a significant reduction of human hair follicle growth. All seven of the PGD₂ analogs tested significantly reduced hair growth. The results are provided in Table III.

**TABLE III**

| **REDUCTION OF HUMAN HAIR FOLLICLE GROWTH BY PGD₂ AND ITS ANALOGS** | | | | |
|---|---|---|---|---|
| | Hair follicle length increase (mm) | | | |
| Prostaglandin | Dose (µM) | Treated | Control | % Reduction |
| PGD₂ | 30 | 0.24 ± 0.10 | 1.89 ± 0.38 | 87.3 ± 5.0 |
| 15-Deoxy-Δ^{12,14}-PGD₂ | 50 | 0.10 ± 0.08 | 1.76 ± 0.27 | 94.3 ± 4.5 |
| 16,16-Dimethyl PGD₂ | 50 | 0.10 ± 0.05 | 1.76 ± 0.27 | 94.3 ± 2.8 |
| 15(S)-15-Methyl PGD₂ | 100 | 0.08 ± 0.06 | 1.10 ± 0.35 | 92.7 ± 5.5 |
| 17-Phenyl trinor PGD₂ | 50 | 0.10 ± 0.12 | 1.62 ± 0.43 | 93.8 ± 7.4 |
| 11-Deoxy-11-methylene PGD₂ | 50 | 1.11 ± 0.14 | 1.76 ± 0.27 | 36.9 ± 8.0 |
| 15(R)-15-Methyl PGD₂ | 50 | 0.04 ± 0.05 | 1.62 ± 0.43 | 97.5 ± 3.1 |

The sequential metabolite of PGD₂, namely, PGJ₂ that is formed in the cells from PGD₂, was also found to reduce hair growth. In addition, natural metabolites and (analogs) of PGJ₂ that were tested reduced hair growth. The results are provided in Table IV.

**TABLE IV**

| Reduction of human hair follicle growth by the PGD₂ metabolite, PGJ₂ and its analogs | | | | |
|---|---|---|---|---|
| | Hair follicle length increase (mm) | | | |
| Prostaglandin | Dose (µM) | Treated | Control | Reduction |
| PGJ₂ | 30 | 0.16 ± 0.25 | 1.89 ± 0.38 | 91.5 ± 13 |
| Δ¹²-PGJ₂ | 30 | 0.29 ± 0.46 | 1.89 ± 0.38 | 84.6 ± 24 |
| 15-Deoxy-Δ^{12,14}-PGJ₂ | 30 | 0.10 ± 0.09 | 1.89 ± 0.38 | 94.7 ± 5.0 |
| 9,10-Dihydro-15-deoxy-^{à 12,14}-PGJ₂ | 50 | 0.02 ± 0.03 | 1.62 ± 0.43 | 98.8 ± 1.9 |

The hair growth inhibition profile by both PGD₂ and PGJ₂ (and its analogs) was found to be dose dependent. The results are provided in Table V.

**TABLE V**

| **DOSE-DEPENDENT REDUCTION OF HUMAN HAIR FOLLICLE GROWTH BY PGD₂ ,PGJ₂ AND ITS METABOLITES** | | | | |
|---|---|---|---|---|
| | Growth of follicle (mm) | | | |
| Prostaglandin | Dose (µM) | Treated | Control | % Reduction |
| PGD₂ | 1 | 1.51 ± 0.28 | 1.58 ± 0.30 | 4.4 |
| | 5 | 1.38 ± 0.30 | 1.58 ± 0.30 | 12.7 |
| | 10 | 0.98 ± 0.32 | 1.58 ± 0.30 | 38.0 |
| PGJ₂ | 1 | 1.43 ± 0.34 | 1.58 ± 0.30 | 9.5 |
| | 5 | 1.03 ± 0.35 | 1.58 ± 0.30 | 34.8 |
| | 10 | 0.50 ± 0.29 | 1.58 ± 0.30 | 68.4 |
| Δ-12-PGJ₂ | 1 | 1.40 ± 0.44 | 1.41 ± 0.34 | 0.7 |
| | 5 | 1.26 ± 0.38 | 1.41 ± 0.34 | 10.6 |
| | 10 | 0.32 ± 0.15 | 1.41 ± 0.34 | 77.3 |
| 15-deoxy-Δ^{12,14}-PGJ₂ | 1 | 1.42 ± 0.37 | 1.41 ± 0.34 | 0 |
| | 5 | 0.21 ± 0.13 | 1.41 ± 0.34 | 34.8 |
| | 10 | 0.08 ± 0.07 | 1.41 ± 0.34 | 94.3 |

Because PGD₂ and PGJ₂ analogs behave as agonists of the PGD₂, and are expected to bind strongly to this receptor, the results indicate that this stimulation of PGD₂ results in reduction of hair growth. These results further demonstrate that activation of PGD₂ by a compound that acts as an agonist of PGD₂ or PGJ₂ results in a reduction of hair growth.

Other embodiments are within the claims.

## Claims

1. A cosmetic method of reducing mammalian hair growth which comprises selecting an area of skin from which reduced hair growth is desired; and applying to said area of skin a dermatologically acceptable composition comprising an agonist of prostaglandin DP-receptor in an amount effective to reduce hair growth.

2. The method of claim 1, wherein said agonist is a prostaglandin D₂ analog.

3. The method of claim 1, wherein said agonist is a prostaglandin D₂ derivative.

4. The method of claim 1, wherein said agonist interacts strongly with the prostaglandin DP-receptor.

5. The method of claim 1, wherein said agonist is 11-deoxy-11-methylene PGD₂**.**

6. The method of claim 1, wherein said agonist is 15(R)-15-methyl PGD₂.

7. The method of claim 1, wherein said agonist is (S)-15-methyl PGD₂.

8. The method of claim 1, wherein said agonist is 15-deoxy-Δ^{12,14}-PGD₂.

9. The method of claim 1, wherein said agonist is 16,16-dimethyl-PGD₂.

10. The method of claim 1, wherein said agonist is 17-phenyl trinor PGD₂.

11. The method of claim 1, wherein said agonist is 9β-halogen-15-cyclohexyl-prostaglandin.

12. The method of claim 1, wherein said agonist is 11α-halogen-15-cyclohexyl-prostaglandin.

13. The method of claim 1, wherein said agonist is acetic acid, [[(2Z)-4-[(1R,2R,3R,5R)-5-chloro-2-[(1E,3S)-3-cyclohexyl-3-L hydroxy-1-propenyl]-3-hydroxycyclopentyl]-2-butenyl]oxy]- (9CI).

14. The method of claim 1, wherein said agonist is butanoic acid, 4-[(1R,2R,3S,6R)-2-[(3S)-3-cyclohexyl-3-hydroxy-1-propynyl]-3-hydroxybicyclo[4.2.0]oct-7-ylidene]-, (4Z)- (9CI).

15. The method of claim 1, wherein said agonist is butanoic acid, 4-[(1S,2S,3R,6S)-2-[(3S)-3-cyclohexyl-3-hydroxy-1-propynyl]-3-hydroxybicyclo [4.2.0]oct-7-ylidene]-, (4Z)- (9CI).

16. The method of claim 1, wherein said agonist is 5-heptenoic acid, 7-[(1S,2S,3S,4R)-3-[(1E,3S)-3-cyclohexyl-3-hydroxy-1-propenyl]-7-oxabicyclo[2.2.1]hept-2-yl]-, (5Z)- (9CI).

17. The method of claim 1, wherein said agonist is 5-heptenoic acid, 7-[(1R,2R,3R,SR)-5-chloro-2-[(1E,3S)-3-cyclohexyl-3-hydroxy-1-propenyl]-3-hydroxycyclopentyl]-, (5Z)- (9CI).

18. The method of claim 1, wherein said agonist is 4-imidazolidineheptanoic acid, 3-[(3R)-3-cyclohexyl-3-hydroxypropyl]-2,5-dioxo-, (4S)-rel- (9CI).

19. The method of claim 1, wherein said agonist is (4R)-(3-[R,S)-3-cyclohexyl-3-hydroxypropyl]-2,5-dioxo)-4-imidazolineheptanoic acid.

20. The method of claim 1, wherein said agonist is benzoic acid, 4-[3-[3-[2-(1-hydroxycyclohexyl)ethyl]-4-oxo-2-thiazolidinyl]propyl]- (9CI).

21. The method of claim 1, wherein said agonist is benzoic acid, 4-[3-[3-(3-hydroxyoctyl)-4-oxo-2-thiazolidinyl]propyl]- (9CI).

22. The method of claim 1, wherein said agonist is 4-imidazolidineheptanoic acid, 3-[(2-cyclohexyl-2-hydroxyethyl)amino]-2,5-dioxo-1-(phenylmethyl)- (9CI).

23. The method of claim 1, wherein said agonist is a PGD₂ metabolite.

24. The method of claim 1, wherein said agonist is 13, 14-dihydro-15-keto PGD_{2.}

25. The method of claim 1, wherein said agonist is PGJ₂.

26. The method of claim 1, wherein said agonist is Δ¹²-PGJ₂.

27. The method of claim 1, wherein said agonist is 15-deoxy-Δ^{12,14}-PGJ₂.

28. The method of claim 1, wherein said agonist is 9,10-dihydro-15-deoxy-Δ^{12,14}-PGJ₂₋

29. The method of claim 1, wherein the concentration of said agonist in said composition is between 0.1% and 30%.

30. The method of claim 1, wherein the composition provides a reduction in hair growth of at least 30% when tested in the Human Hair Follicle assay,

31. The method of claim 1, wherein the composition provides a reduction in hair growth of at least 60% when tested in the Human Hair Follicle assay.

32. The method of claim 1, wherein the agonist is applied to the skin in an amount of from 10 to 3000 micrograms of said agonist per square centimeter of skin.

33. The method of claim 1, wherein said area of skin is on the face of a human.

34. The method of claim 1, wherein the composition is applied to the area of skin in conjunction with shaving.

35. The method of claim 1, wherein said area of skin is on a leg of the human.

36. The method of claim 1, wherein said area of skin is on an arm of the human.

37. The method of claim 1, wherein said area of skin is in an armpit of the human.

38. The method of claim 1, wherein said area of skin is on the torso of the human.

39. The method of claim 1, wherein the composition is applied to an area of skin of a woman with hirsutism.

40. The method of claim 1, wherein said hair growth comprises androgen stimulated hair growth.

41. The method of claim 1, wherein the composition further includes a second component that also causes a reduction in hair growth.

42. A cosmetic method of reducing mammalian hair growth, which comprises selecting an area of skin including hair follicles from which reduced hair growth is desired; and
applying to the skin a compound selected from the group consisting of prostaglandin D₂, analogs of prostaglandin D₂, PGJ₂, or an analog of PGJ₂, in an amount effective to reduce hair growth.

43. Use of an agonist of prostaglandin DP-receptor for preparation of a medicament for reducing mammalian hair growth.

44. Cosmetic use of a composition comprising an agonist of prostaglandin DP-receptor for reducing unwanted hair growth.

## Patentansprüche

1. Kosmetisches Verfahren zum Reduzieren von Haarwuchs bei Säugern, das das Auswählen eines Hautbereichs umfasst, bei dem reduzierter Haarwuchs gewünscht wird; und Auftragen einer hautverträglichen Zusammensetzung, die einen Agonisten von Prostaglandin-DP-Rezeptor in einer zum Reduzieren des Haarwuchses wirksamen Menge umfasst, auf den Hautbereich.

2. Verfahren nach Anspruch 1, wobei der Agonist ein Prostaglandin-D₂-Analog ist.

3. Verfahren nach Anspruch 1, wobei der Agonist ein Prostaglandin-D₂-Derivat ist.

4. Verfahren nach Anspruch 1, wobei der Agonist stark mit dem Prostaglandin-DP-Rezeptor interagiert.

5. Verfahren nach Anspruch 1, wobei der Agonist 11-Deoxy-11-methylen-PGD₂ ist.

6. Verfahren nach Anspruch 1, wobei der Agonist 15(R)-15-Methyl-PGD2 ist.

7. Verfahren nach Anspruch 1, wobei der Agonist (S)-15-Methyl PGD₂ ist.

8. Verfahren nach Anspruch 1, wobei der Agonist 15-Deoxy-Δ^{12,14}-GD₂ ist.

9. Verfahren nach Anspruch 1, wobei der Agonist 16,16-Dimethyl-PGD2 ist.

10. Verfahren nach Anspruch 1, wobei der Agonist 17-Phenyltrinor-PGD2 ist.

11. Verfahren nach Anspruch 1, wobei der Agonist 9β-Halogen-15-cyclo-hexylprostaglandin ist.

12. Verfahren nach Anspruch 1, wobei der Agonist 11αHalogen-15-cyclo hexylprostaglandin ist.

13. Verfahren nach Anspruch 1, wobei der Agonist Essigsäure-[[(2Z)-4-[(1R,2R,3R,5R)-5-chlor-2-[(1E,3S)-3-cyclohexyl-3-L hydroxy-1-propenyl]-3-hydroxycyclopentyl]-2-butenyl]oxy]-(9CI) ist.

14. Verfahren nach Anspruch 1, wobei der Agonist Buttersäure-4-[(1R,2R,3S,6R)-2-[(3S)-3-cyclohexyl-3-hydroxy-1-propynyl]-3-hydroxybicyc-lo[4.2.0]oct-7-yliden]-, (4Z)- (9CI) ist.

15. Verfahren nach Anspruch 1, wobei der Agonist Buttersäure-4-[1S,2S,3R,6S)-2-[(3S)-3-cyclohexyl-3-hydroxy-1-propynyl]-3-bydroxybicyclo [4.2.0]oct-7-yliden]-, (4Z)- (9CI) ist.

16. Verfahren nach Anspruch 1, wobei der Agonist 5-Heptensäure-7-[(1S,2S,3S,4R)-3-[(1E,3S)-3-cyclohexyl-3-hydroxy-1-propenyl]-7-oxabicyclo-[2.2.1] hept-2-yl]-, (5Z)- (9CI) ist.

17. Verfahren nach Anspruch 1, wobei der Agonist 5-Heptensäure-7-[(1R,2R,3R,SR)-5-chlor-2-[(1E,3S)-3-cyclohexyl-3-hydroxy-1-propenyl]-3-hydroxycyclopentyl]-, (5Z)- (9CI) ist.

18. Verfahren nach Anspruch 1, wobei der Agonist 4-Imidazolidinheptan-säure-3-[(3R)-3-cyclohexyl-3-hydroxypropyl]-2,5-dioxo-, (4S)-rel- (9CI) ist.

19. Verfahren nach Anspruch 1, wobei der Agonist (4R)-(3-[R,S)3-Cyclohexyl-3-hydroxypropyl]-2,5-dioxo)-4-imidazolinheptansäure ist.

20. Verfahren nach Anspruch 1, wobei der Agonist Benzoesäure-4-[3-[3-[2-(1-hydroxycyclohexyl)ethyl]-4-oxo-2-thiazolidinyl]propyl]-(9ÑI) ist.

21. Verfahren nach Anspruch 1, wobei der Agonist Benzoesäure-4-[3-[3-(3-hydroxyoctyl)-4-oxo-2-thiazolidinyl]propyl]-(9CI) ist.

22. Verfahren nach Anspruch 1, wobei der Agonist 4-Imidazolidinheptansäure-3-[(2-cyclohexyl-2-hydroxyethyl)amino]-2,5-dioxo-1-(phenylmethyl)-(9CI) ist.

23. Verfahren nach Anspruch 1, wobei der Agonist ein PGD₂-Metabolit ist.

24. Verfahren nach Anspruch 1, wobei der Agonist 13,14-Dihydro-15-keto-PGD₂ ist.

25. Verfahren nach Anspruch 1, wobei der Agonist PGJ₂ ist.

26. Verfahren nach Anspruch 1, wobei der Agonist Δ¹² -PGJ₂ ist.

27. Verfahren nach Anspruch 1, wobei der Agonist 15-Deoxy-Δ ^{12,14}-PGJ₂ ist.

28. Verfahren nach Anspruch 1, wobei der Agonist 9,10-Dihydro-15-deoxy-Δ^{12,14}-PGJ₂ ist.

29. Verfahren nach Anspruch 1, wobei die Konzentration des Agonisten in der Zusammensetzung zwischen 0,1 % und 30 % liegt.

30. Verfahren nach Anspruch 1, wobei die Zusammensetzung eine Reduzierung des Haarwuchses von mindestens 30 % bereitstellt, wenn mit dem Nachweisverfahren für menschliche Haarfollikel getestet wird.

31. Verfahren nach Anspruch 1, wobei die Zusammensetzung eine Reduzierung des Haarwuchses von mindestens 60 % bereitstellt, wenn mit dem Nachweisverfahren für menschliche Haarfollikel getestet wird.

32. Verfahren nach Anspruch 1, wobei der Agonist in einer Menge von 10 bis 3000 Mikrogramm Agonist pro Quadratzentimeter Haut auf die Haut aufgetragen wird.

33. Verfahren nach Anspruch 1, wobei der Hautbereich auf dem Gesicht eines Menschen ist.

34. Verfahren nach Anspruch 1, wobei die Zusammensetzung in Verbindung mit Rasieren auf den Hautbereich aufgetragen wird.

35. Verfahren nach Anspruch 1, wobei der Hautbereich auf einem Bein des Menschen ist.

36. Verfahren nach Anspruch 1, wobei der Hautbereich auf einem Arm des Menschen ist.

37. Verfahren nach Anspruch 1, wobei der Hautbereich in einer Achselhöhle des Menschen ist.

38. Verfahren nach Anspruch 1, wobei der Hautbereich auf dem Rumpf des Menschen ist.

39. Verfahren nach Anspruch 1, wobei die Zusammensetzung auf einen Hautbereich einer Frau mit Hirsutismus aufgetragen wird.

40. Verfahren nach Anspruch 1, wobei der Haarwuchs androgen stimulierten Haarwuchs umfasst.

41. Verfahren nach Anspruch 1, wobei die Zusammensetzung ferner einen zweiten Bestandteil enthält, der auch eine Reduzierung des Haarwuchses verursacht.

42. Kosmetisches Verfahren zum Reduzieren von Haarwuchs bei Säugern, das das Auswählen eines Hautbereichs mit Haarfollikeln, bei dem reduzierter Haarwuchs gewünscht wird, umfasst; und
Auftragen einer Verbindung, ausgewählt aus der Gruppe, bestehend aus Prostaglandin-D₂, Analogen von Prostaglandin-D₂, PGJ₂ oder einem Analog von PGJ₂, in einer zum Reduzieren des Haarwuchses wirksamen Menge auf die Haut.

43. Verwendung eines Agonisten von Prostaglandin-DP-Rezeptor zur Herstellung eines Medikaments zum Reduzieren von Haarwuchs bei Säugern,

44. Kosmetische Verwendung einer Zusammensetzung, die einen Agonisten von Prostaglandin-DP-Rezeptor umfasst zum Reduzieren unerwünschten Haarwuchses.

## Revendications

1. Procédé cosmétique de réduction de la pousse des poils de mammifères qui comprend le choix d'une zone de peau de laquelle une pousse des poils réduite est souhaitée ; et l'application sur ladite zone de peau d'une composition acceptable du point de vue dermatologique comprenant un agoniste de récepteur de prostaglandine DP en une quantité efficace pour réduire la pousse des poils.

2. Procédé selon la revendication 1, dans lequel ledit agoniste est un analogue de prostaglandine D₂.

3. Procédé selon la revendication 1, dans lequel ledit agoniste est un dérivé de prostaglandine D₂.

4. Procédé selon la revendication 1, dans lequel ledit agoniste interagit fortement avec le récepteur de prostaglandine DP.

5. Procédé selon la revendication 1, dans lequel ledit agoniste est le 11-déoxy-11-méthylène PGD₂.

6. Procédé selon la revendication 1, dans lequel ledit agoniste est le 15(R)-15-méthyl PGD₂.

7. Procédé selon la revendication 1, dans lequel ledit agoniste est le (S)-15-méthyl PGD₂.

8. Procédé selon la revendication 1, dans lequel ledit agoniste est le 15-déoxy-Δ^{2,14}-PGD₂.

9. Procédé selon la revendication 1, dans lequel ledit agoniste est le 16,16-diméthyl-PGD₂.

10. Procédé selon la revendication 1, dans lequel ledit agoniste est le 17-phényl trinor PGD₂.

11. Procédé selon la revendication 1, dans lequel ledit agoniste est la 9β-halogène-15-cyclohexyl-prostaglandine.

12. Procédé selon la revendication 1, dans lequel ledit agoniste est la 11α-halogène-15-cyclohexyl-prostaglandine.

13. Procédé selon la revendication 1, dans lequel ledit agoniste est l'acide acétique, [[(2Z)-4-[(1R,2R,3R,5R)-5-chloro-2-[(1E,3S)-3-cyclohexyl-3-L hydroxy-1-propényl]-3-hydroxycyclopentyl]-2-butenyl]oxy]-(9CI).

14. Procédé selon la revendication 1, dans lequel ledit agoniste est l'acide butanoïque, 4-[(1R,2R,3S,6R)-2-[(3S)-3-cyclohexyl-3-hydroxy-1-propynyl]-3-hydroxybicyclo[4.2.0]oct-7-ylidène]-, (4Z)- (9CI).

15. Procédé selon la revendication 1, dans lequel ledit agoniste est l'acide butanoïque, 4-[(1S,2S,3R,6S)-2-[(3S)-3-cyclohexyl-3-hydroxy-1-propynyl]-3-hydroxybicyclo [4.2.0]oct-7-ylidène]-, (4Z)- (9CI).

16. Procédé selon la revendication 1, dans lequel ledit agoniste est l'acide 5-hepténoïque, 7-[(1S,2S,3S,4R)-3-[(1E,3S)-3-cyclohexyl-3-hydroxy-1 -propényl]-7-oxabicyclo[2.2.1] hept-2-yl]-, (5Z)- (9CI).

17. Procédé selon la revendication 1, dans lequel ledit agoniste est l'acide 5-hepténoïque, 7-[(1R,2R,3R,SR)-S-chloro-2-[(1E,3S)-3-cyclohexyl-3-hydroxy-1-propényl]-3-hydroxycyclopentyl]-, (5Z)- (9CI).

18. Procédé selon la revendication 1, dans lequel ledit agoniste est l'acide 4-imidazolidineheptanoïque, 3-[(3R)-3-cyclohexyl-3-hydroxypropyl]-2,5-dioxo-, (4S)-rel- (9CI).

19. Procédé selon la revendication 1, dans lequel ledit agoniste est l'acide (4R)-(3-[R,S)3-cyclohexyl-3-hydroxypropyl]-2,5-dioxo)-4-imidazolineheptanoïque.

20. Procédé selon la revendication 1, dans lequel ledit agoniste est l'acide benzoïque, 4-[3-[3-[2-(1-hydroxycyclohexyl)éthyl]-4-oxo-2-thiazolidinyl]propyl]- (9CI).

21. Procédé selon la revendication 1, dans lequel ledit agoniste est l'acide benzoïque, 4-[3-[3-(3-hydroxyoctyl)-4-oxo-2-thiazolidinyl]propyl]- (9CI).

22. Procédé selon la revendication 1, dans lequel ledit agoniste est l'acide 4-imidazolidineheptanoïque, 3-[(2-cyclohexyl-2-hydroxyéthyl)amino]-2,5-dioxo-1-(phénylméthyl)-(9CI).

23. Procédé selon la revendication 1, dans lequel ledit agoniste est un métabolite de PGD₂.

24. Procédé selon la revendication 1, dans lequel ledit agoniste est le 13, 14-dihydro-15-céto PGD₂.

25. Procédé selon la revendication 1, dans lequel ledit agoniste est le PGJ₂.

26. Procédé selon la revendication 1, dans lequel ledit agoniste est le Δ¹² -PGJ₂.

27. Procédé selon la revendication 1, dans lequel ledit agoniste est 15-déoxy-Δ^{12,14}-PGJ₂.

28. Procédé selon la revendication 1, dans lequel ledit agoniste est le 9,10-dihydro-15-deoxy-Δ^{12,14} -PGJ₂.

29. Procédé selon la revendication 1, dans lequel la concentration dudit agoniste dans ladite composition est entre 0,1 % et 30%.

30. Procédé selon la revendication 1, dans lequel la composition fournit une réduction de pousse des poils d'au moins 30 % lorsqu'elle est testée dans l'essai de follicule pileux humain.

31. Procédé selon la revendication 1, dans lequel la composition fournit une réduction de pousse des poils d'au moins 60 % lorsqu'elle est testée dans l'essai de follicule pileux humain.

32. Procédé selon la revendication 1, dans lequel l'agoniste est appliqué sur la peau en une quantité de 10 à 3000 microgrammes dudit agoniste par centimètre carré de peau.

33. Procédé selon la revendication 1, dans lequel ladite zone de peau est sur le visage d'un humain.

34. Procédé selon la revendication 1, dans lequel la composition est appliquée sur la zone de peau conjointement avec le rasage.

35. Procédé selon la revendication 1, dans lequel ladite zone de peau est sur une jambe de l'humain.

36. Procédé selon la revendication 1, dans lequel ladite zone de peau est sur un bras de l'humain.

37. Procédé selon la revendication 1, dans lequel ladite zone de peau est dans une aisselle de l'humain.

38. Procédé selon la revendication 1, dans lequel ladite zone de peau est sur le torse de l'humain.

39. Procédé selon la revendication 1, dans lequel la composition est appliquée sur une zone de peau d'un femme atteinte d'hirsutisme.

40. Procédé selon la revendication 1, dans lequel ladite pousse des poils comprend une pousse des poils stimulée par des androgènes.

41. Procédé selon la revendication 1, dans lequel la composition inclut, en outre, un deuxième composant qui provoque également une réduction de pousse des poils.

42. Procédé cosmétique de réduction de la pousse des poils de mammifères, qui comprend le choix d'une zone de peau incluant des follicules pileux desquels une pousse des poils réduite est souhaitée ; et
l'application sur la peau d'un composé choisi dans le groupe constitué de prostaglandine D₂, analogues de prostaglandine D₂, PGJ₂, ou un analogue de PGJ₂, en une quantité efficace pour réduire la pousse des poils.

43. Utilisation d'un agoniste de récepteur de prostaglandine DP pour la préparation d'un médicament pour réduire la pousse des poils de mammifères,

44. Utilisation cosmétique d'une composition comprenant un agoniste de récepteur de prostaglandine DP pour réduire une pousse indésirable des poils.
